# EUROPÄISCHE PATENTSCHRIFT

(11) **EP 3 129 137 B1**
(45) Veröffentlichungstag und Bekanntmachung des Hinweises auf die Patenterteilung: **22.01.2020**
(21) Anmeldenummer: 15711220.2
(22) Anmeldetag: 23.03.2015
(51) Int. Cl.: B01J 23/46, C07C 67/10

(54) **VERFAHREN ZUR DARSTELLUNG EINER AKTIVEN RUTHENIUM-KATALYSATORLÖSUNG FÜR DIE UMVINYLIERUNG VON CARBONSÄUREN**
METHOD FOR PROVIDING AN ACTIVE RUTHENIUM CATALYST SOLUTION FOR THE TRANSVINYLATION OF CARBOXYLIC ACIDS
PROCÉDÉ DE PRÉPARATION D'UNE SOLUTION CATALYTIQUE ACTIVE DE RUTHÉNIUM POUR LA VINYLISATION D'ACIDES CARBOXYLIQUES

(30) Priorität: 10.04.2014 DE 102014206915
(43) Veröffentlichungstag der Anmeldung: 15.02.2017
(73) Patentinhaber: Wacker Chemie AG, 81737 München (DE)
(72) Erfinder: GIGLER, Peter, 85221 Dachau (DE)
(74) Vertreter: Schuderer, Michael
(86) Internationale Anmeldenummer: PCT/EP2015/056099
(87) Internationale Veröffentlichungsnummer: WO 2015/154978

(56) Entgegenhaltungen:
- US-A- 5 155 253

## Beschreibung

Die Erfindung betrifft ein Verfahren zur Darstellung einer aktiven Ru-Katalysatorlösung für die Umvinylierung von Carbonsäuren.

Die Umvinylierung von Carbonsäuren dient der Darstellung von Vinylestern. Darunter versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

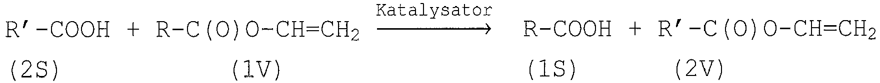

Aus der EP 376075 B1 ist die Umvinylierung von Vinylestern mit Carbonsäuren in Gegenwart von Palladiumkatalysator bekannt, wobei Kupferbromid und spezielle Lithiumverbindungen als Cokatalysatoren eingesetzt werden.

Neben Palladium-Katalysatoren und Quecksilber-Katalysatoren werden im Stand der Technik zur Umvinylierung von Vinylestern mit Carbonsäuren auch Ruthenium-Verbindungen als Katalysator eingesetzt. Ruthenium-Verbindungen zeichnen sich durch ihre hohe Löslichkeit, geringe Flüchtigkeit und hohe thermische Stabilität aus. Hinzu kommt eine hohe, Temperatur-induzierbare Aktivität.

In der EP 351603 A2 (= EP506070, US4981973, US 5155253) wird ein Verfahren zur Umvinylierung von Carbonsäuren unter Verwendung verschiedener Ru-Verbindungen als Katalysator-Precursor beschrieben. Die Autoren postulieren eine [Ru(CO)₂RCO₂]-Einheit als entscheidendes Strukturelement bei der Bildung der aktiven Spezies. Demzufolge können alle Ru-Verbindungen als Katalysatorvorstufen eingesetzt werden, die sich in situ in dieses Strukturelement umwandeln lassen. Bei der Verwendung von Ruthenium(III)chlorid als Ausgangsverbindung ist die Zugabe eines Alkali-Carboxylats erforderlich, um die aktive Spezies zu generieren. Beispiel 16 beschreibt die Umvinylierung von Benzoesäure (100 mmol) mit Vinylacetat (200 mmol) unter Einsatz eines RuCl₃/Natriumacetat-Gemisches, welches unter den Umvinylierungsbedingungen in situ zur aktiven Katalysatorverbindung umgewandelt wird. Bei der Umvinylierung wird nach einer Stunde Reaktionszeit bei 130°C eine Ausbeute von 27 % Vinylbenzoat erreicht.

In Adv. Synth. Catal. 2013, 355, 2845 - 2859 wird die Theorie der EP 351603 A2 bestätigt und ein [Ru(CO)₃(RCO₂)₂]-Komplex als aktive Katalysatorspezies postuliert. Die Bildung der katalytisch aktiven Spezies erfolgt durch Umsetzung von RuCl₃ mit Natriumhydroxid, Vinylacetat (Eduktvinylester) und Propionsäure (Eduktcarbonsäure). Die Umsetzung erfolgt über 4 Stunden bei einer Temperatur von 140°C und einem molaren Verhältnis von Vinylacetat zu Propionsäure von 2,7 : 1. Die Ausbeute an aktivem Ruthenium-Katalysator wird mit 53 % angegeben.

Die EP 497340 A2 (US5210207) beschreibt ein Umvinylierungsverfahren zur Darstellung von Produktvinylestern, deren Siedepunkte höher liegen als die der Eduktvinylester. Durch Reaktivdestillation von mindestens einer der Produktkomponenten wird das Gleichgewicht der Reaktion auf die Produktseite verschoben. Bevorzugt werden hierfür die in EP 351603 A2 beschriebenen Ru-Katalysatoren eingesetzt. Beispiele in denen die aktive Katalysatorspezies aus RuCl₃ generiert und eingesetzt werden, sind nicht angeführt. Es werden als aktive Ru-Katalysatoren Ru-Carbonyl und Ru-Dicarbonyl-Acetat eingesetzt.

In der WO 92/09554 A1 wird ein Verfahren beschrieben, bei dem die Reaktionsmasse nach der Umvinylierung zuerst separiert und der Produktvinylester anschließend durch Azeotropdestillation abgetrennt wird. Dieses Verfahren zielt vor allem auf die Trennung von Säure/Vinylester-Gemischen mit geringen Siedepunktdifferenzen. In der Umvinylierungsreaktion werden bevorzugt die Ru-Katalysatoren aus der EP 351603 A2 eingesetzt (Ru-Carbonyl und Ru-Dicarbonyl-Acetat). Der Einsatz RuCl₃-basierter Katalysatorsysteme wird in den Beispielen nicht beschrieben.

Die WO 2013/117294 A1 beschreibt ein kontinuierliches Verfahren zur Darstellung von Carbonsäurevinylestern. Die übergangsmetallkatalysierte Umvinylierung wird im stationären Zustand betrieben und das Reaktionsgemisch in einem Folgeschritt aufgetrennt. WO 2013/117295 beschreibt eine weitere Ausgestaltung dieses Verfahrens mit einer nachträglichen Derivatisierung der entstehenden, konjugaten Säure des Eduktvinylesters. In den Beispielen beider Schriften werden hauptsächlich Pd-Katalysatoren für die Umvinylierung eingesetzt. RuCl₃-basierte Katalysatoren werden nicht beschrieben.

Die Verwendung von Ru-Katalysatoren in der Umvinylierungsreaktion birgt deutliche Vorteile gegenüber Pd-Katalysatoren hinsichtlich Löslichkeit, Flüchtigkeit, thermische Stabilität und thermisch induzierbarer Aktivität. Zahlreiche Ru-Verbindungen lassen sich in situ zu aktiven Ru-Spezies umwandeln, welche die Umvinylierungsreaktion katalysieren. Die gezielte Herstellung einer konzentrierten, aktiven Katalysatorlösung auf Basis industriell verfügbarer Rutheniumhalogenide ist nicht bekannt.

Es bestand daher die Aufgabe, ein Verfahren zur Darstellung einer aktiven Ru-Katalysatorlösung mit einer Ru-Konzentration von mehr als 0,5 % zu entwickeln, das sich durch eine hohe Ru-Ausbeute bezogen auf das eingesetzte Rutheniumhalogenid und eine hohe Aktivität auszeichnet.

Gegenstand der Erfindung ist ein Verfahren zur Darstellung einer aktiven Ruthenium-Katalysatorlösung, zur Umvinylierung eines Eduktvinylesters mit einer Eduktcarbonsäure, mit einer Ru-Konzentration größer als 0,5 Gew.-%, Ru-Metall bezogen auf das Gesamtgewicht der Ruthenium-Katalysatorlösung, dadurch gekennzeichnet, dass
a) mindestens ein Ruthenium(III)halogenid mit mindestens einer anorganischen oder organischen Base, mit mindestens einem Eduktvinylester und mit mindestens einer Eduktcarbonsäure bei einer Temperatur von 70°C bis 170°C umgesetzt wird, wobei
b) das molare Verhältnis von Eduktvinylester zu Eduktcarbonsäure 1 : 1,8 bis 1,8 : 1 beträgt, und
c) das Ruthenium(III)halogenid in einer Menge von ≥ 0,5 Gew.-% an Ru-Metall bezogen auf das Gesamtgewicht von Eduktvinylester und Eduktcarbonsäure eingesetzt wird.

Unter einer aktiven Ruthenium-Katalysatorlösung wird dabei eine Lösung von einer oder mehreren Ruthenium-Verbindungen verstanden, welche ohne zusätzlichen Formierungsschritt die Umvinylierung eines Eduktvinylesters mit einer Eduktcarbonsäure katalysiert.

Als Ruthenium(III)halogenide können Ruthenium(III)chlorid, Ruthenium(III)bromid und Ru(III)iodid eingesetzt werden. Bevorzugt erfolgt der Einsatz von Ruthenium(III)chlorid. Das Ru(III)-halogenid wird typischerweise in Konzentrationen von > 0,5 Gew.-% (Gehalt Ruthenium bezogen auf die Reaktionsmasse aus Eduktvinylester und Eduktcarbonsäure) eingesetzt. Die Obergrenze beträgt vorzugsweise 4 Gew.-% (Gehalt Ruthenium bezogen auf die Reaktionsmasse aus Eduktvinylester und Eduktcarbonsäure). Besonders bevorzugt werden Konzentration von 0,75 Gew.-% bis 3 Gew.-%, am meisten bevorzugt sind Konzentrationen von 1 Gew.-% bis 2 Gew.-%, jeweils Gehalt Ruthenium bezogen auf die Reaktionsmasse aus Eduktvinylester und Eduktcarbonsäure.

Als Basen können anorganische Basen wie Hydroxide, Carbonate und Hydrogencarbonate der Alkali- und Erdalkalimetalle, Ammoniak sowie organische Basen wie Carboxylate und Alkoholate der Alkali- und Erdalkalimetalle und organische Amine verwendet werden. Bevorzugt werden Hydroxide und Carboxylate der Alkali- und Erdalkalimetalle eingesetzt. Beispiele hierfür sind NaOH, KOH, Na-Acetat, K-Acetat. Besonders bevorzugt erfolgt der Einsatz von Natriumhydroxid. Im Allgemeinen werden jeweils 1 bis 10 Mol-Äquivalente Base pro Mol Ruthenium(III)halogenid eingesetzt. Bevorzugt ist die Verwendung von 2 bis 5 Mol-Äquivalenten, besonders bevorzugt sind 3 Mol-Äquivalente.

Als Vinylester können beliebige Carbonsäurevinylester der allgemeinen Formel R-C(O)O-CH=CH₂ eingesetzt werden, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann. Bevorzugt ist die Verwendung niedermolekularer Eduktvinylester, wobei R ein Alkylrest mit 1 bis 6 C-Atomen ist, beispielsweise Vinylacetat, Vinylpropionat und Vinylpivalat. Besonders bevorzugt ist die Verwendung von Vinylacetat.

Ferner wird der Reaktion mindestens eine Eduktcarbonsäure der allgemeinen Formel R'-COOH zugeführt, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann. Bevorzugt werden Eduktcarbonsäuren der genannten Verbindungsklassen mit 2 bis 18 C-Atomen eingesetzt. Beispiele hierfür sind Essigsäure, Propionsäure, n-Buttersäure, iso-Buttersäure, n-Valeriansäure, 2-Methylbuttersäure, 3-Methylbuttersäure, Pivalinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure, Laurinsäure, Myristinsäure, Palmitinsäure, Stearinsäure, Benzoesäure, Naphthalincarbonsäure. Besonders bevorzugt werden Versaticsäuren^{R} (alpha-verzweigte Carbonsäuren mit 9 bis 12 C-Atomen der Fa. Momentive) oder Neo-Säuren mit 9 bis 12 C-Atomen und Fettsäuren wie Laurinsäure, Myristinsäure, Palmitinsäure, Stearinsäure. Das molare Verhältnis von Eduktvinylester zu Eduktcarbonsäure beträgt 1 : 1,8 bis 1,8 : 1. Bevorzugt ist ein Verhältnis von Eduktvinylester zu Eduktcarbonsäure von 1,5 : 1 bis 1 : 1, besonders bevorzugt ist ein Verhältnis von circa 1 : 1.

Den genannten Edukten kann gegebenenfalls ein Polymerisationsinhibitor zugesetzt werden. Bevorzugt werden 100 bis 10000 ppm, bezogen auf die Reaktionsmasse aus Eduktvinylester und Eduktcarbonsäure, Polymerisationsinhibitor eingesetzt. Beispiele für Polymerisationsinhibitoren sind Hydrochinon, Methoxyhydrochinon, tertiär-Butylcatechol, Phenothiazin oder Nitroxidradikale wie TEMPO oder 4-OH-TEMPO (TEMPO = 2,2,6,6-Tetramethylpiperidinyloxyl). Bevorzugt ist die Verwendung von Phenothiazin oder Hydrochinon.

Die Edukte können einzeln oder als Gemisch zugegeben werden, und in einem oder mehreren Schritten bei einer Temperatur von 70°C bis 170°C zur Reaktion gebracht werden.

In einer bevorzugten Ausführungsform werden Base, Eduktcarbonsäure und gegebenenfalls Polymerisationsinhibitor im Reaktor bei einer Temperatur von vorzugsweise 80°C bis 160°C, besonders bevorzugt von 120°C bis 140°C, und einem Druck von vorzugsweise kleiner oder gleich 1 bar abs. für vorzugsweise 0,5 h bis 3 h, besonders bevorzugt 1 h vorbehandelt. Entstehendes Reaktionswasser kann gegebenenfalls abgedampft oder im Vakuum entfernt werden. Anschließend kann das Ru(III)halogenid zugegeben werden, und die Reaktion wird vorzugsweise unter gleichen Druck- und Temperaturbedingungen für vorzugsweise weitere 0,25 h bis 2 h, besonders bevorzugt 0,5 h bis 1 h fortgesetzt. Vorzugsweise wird anschließend der Eduktvinylester zugegeben und bei einer Temperatur von 70°C bis 170°C, vorzugsweise bei einer Temperatur von 120°C bis 150°C der Druck im Allgemeinen auf > 1 bar abs. erhöht, und die Reaktion unter diesen Bedingungen für 8 h bis 16 h fortgesetzt. Die Reaktion wird bevorzugt in einer Schutzgasatmosphäre, beispielsweise Stickstoff, in an sich bekannter Weise, durchgeführt. Die Reaktionsdauer beträgt bei dem erfindungsgemäßen Verfahren im Allgemeinen insgesamt 1 bis 24 Stunden, vorzugsweise 8 bis 20 Stunden, besonders bevorzugt 12 bis 17 Stunden.

Nach der Reaktion kann die Abtrennung ungelöster Bestandteile durch Filtration, Extraktion, Sedimentation oder Fällung erfolgen. Bevorzugt werden ungelöste Bestandteile durch Filtration abgetrennt.

Gegebenenfalls kann die damit erhaltene Ru-Katalysatorlösung durch Destillation weiter aufkonzentriert werden.

Die Teilschritte des Verfahrens, sowohl die Reaktion als auch die Aufarbeitungsschritte, können diskontinuierlich, semikontinuierlich und vollkontinuierlich durchgeführt werden. Das Verfahren wird bevorzugt diskontinuierlich durchgeführt.

Mit dem erfindungsgemäßen Verfahren können aktive Ru-Katalysatorlösungen mit einer Konzentrationen von Ruthenium, in katalytisch aktiver und löslicher Form, von größer als 0,5 Gew.-%, bezogen auf das Gesamtgewicht der Lösung, hergestellt werden. Überraschend wurde gefunden, dass bei einem Vinylester zu Carbonsäure-Verhältnis im beanspruchten Bereich von 1 : 1,8 bis 1,8 : 1 mehr als 80 Gew.-% des eingesetzten Rutheniums in eine lösliche und aktive Form gebracht werden kann.

Das erfindungsgemäße Verfahren ermöglicht somit die Darstellung einer aktiven, hochkonzentrierten Katalysatorlösung (größer als 0,5 Gew.-%, Gehalt Ruthenium bezogen auf eine Mischung (Lösungsmittel), die im Wesentlichen aus Produktvinylester, der konjugaten Säure des Eduktvinylesters, Eduktvinylester und Eduktcarbonsäure besteht), auf Basis kommerziell verfügbarer Rutheniumhalogenide. Die Initiierungsphase, wie sie beim Einsatz von Katalysatorvorstufen auftritt, kann durch den Einsatz solcher präformierter Katalysatorlösungen vermieden werden.

Nicht beansprucht ist die Verwendung der erfindungsgemäßen Ru-Katalysatorlösung in der Umvinylierung einer Eduktcarbonsäure mit einem Eduktvinylester zu einem Produktvinylester und der korrespondierenden Säure des Eduktvinylesters. Solche Verfahren zur Umvinylierung von Carbonsäuren mittels Ruthenium-Katalyse sind dem Fachmann bekannt, beispielsweise aus den Offenlegungssschriften DE 102013224491 und DE 102013224496.

### Beispiele:

Die nachfolgenden Beispiele dienen der näheren Erläuterung der Erfindung.

Die Ru-Ausbeute berechnet sich gemäß A (%) = 100 x m_{Ru}(F)/ (m_{Ru}(F) + m_{Ru}(FK)), wobei m_{Ru}(F) die Masse des Rutheniums im Filtrat und m_{Ru}(FK) die Masse des Rutheniums im Filterkuchen darstellt.

### Vergleichsbeispiel 1:

### Herstellung einer Katalysatorlösung mit niedriger Ru-Konzentration (< 0,5 Gew.-%) und einem Vinylester/Säure-Verhältnis von 2 : 1.

In einem 100 ml Berghoff-Autoklav wurden 38,5 g (0,19 mol) Laurinsäure, 0,26 g (6,5 mmol) Natriumhydroxid, 0,07 g Phenothiazin gegeben und 1 Stunde lang auf 120°C erhitzt, sodass entstehendes Reaktionswasser entweichen konnte. Anschließend wurden 0,60 g (2 mmol) Ruthenium(III)chlorid-Hydrat zugegeben und eine weitere halbe Stunde auf 120°C erhitzt, sodass Wasser entweichen konnte. Nach Zugabe von 33,0 g (0,38 mol) Vinylacetat bei 60°C wurde die Mischung 12 Stunden bei 1,6 bar abs. auf 120°C erhitzt. Nach dem Abkühlen wurde die Reaktionsmischung bei 60°C filtriert (m_{Ru}(FK) = 0,024 g) und mit 20 g Vinylacetat gewaschen, wonach man 83,0 g eines rot-braunen Filtrats mit einem Ru-Gehalt von 0,24 Gew.-%, bezogen auf die Gesamtmasse des Filtrats, erhielt.

### Vergleichsbeispiel 2:

### Herstellung einer Katalysatorlösung mit niedriger Ru-Konzentration (< 0,5 Gew.-%) und einem Vinylester/Säure-Verhältnis von 1 : 1

In einem 100 ml Berghoff-Autoklav wurden 50,0 g (0,25 mol) Laurinsäure, 0,26 g (6,5 mmol) Natriumhydroxid, 0,07 g Phenothiazin gegeben und 1 Stunde lang auf 120°C erhitzt, sodass entstehendes Reaktionswasser entweichen konnte. Anschließend wurden 0,60 g (2 mmol) Ruthenium(III)chlorid-Hydrat zugegeben und eine weitere halbe Stunde auf 120°C erhitzt, sodass Wasser entweichen konnte. Nach Zugabe von 21,5 g (0,25 mol) Vinylacetat bei 60°C wurde die Mischung 12 Stunden bei 1,2 bar abs. auf 120°C erhitzt. Nach dem Abkühlen wurde die Reaktionsmischung bei 60°C filtriert (m_{Ru}(FK) = 0,017 g) und mit 20 g Vinylacetat gewaschen, wonach man 81,4 g eines rot-braunen Filtrats mit einem Ru-Gehalt von 0,28 Gew.-% erhielt.

### Vergleichsbeispiel 3:

### Herstellung einer Katalysatorlösung mit hoher Ru-Konzentration (> 0,5 Gew.-%) und einem Vinylester/Säure-Verhältnis von 2 : 1

In einem 100 ml Berghoff-Autoklav wurden 38,5 g (0,19 mol) Laurinsäure, 1,02 g (25,5 mmol) Natriumhydroxid, 0,07 g Phenothiazin gegeben und 1 Stunde lang auf 120°C erhitzt, sodass entstehendes Reaktionswasser entweichen konnte. Anschließend wurden 2,36 g (8,4 mmol) Ruthenium(III)chlorid-Hydrat zugegeben und eine weitere halbe Stunde auf 120°C erhitzt, sodass Wasser entweichen konnte. Nach Zugabe von 33,0 g (0,38 mol) Vinylacetat bei 60°C wurde die Mischung 12 Stunden bei 2,1 bar abs. auf 120°C erhitzt. Nach dem Abkühlen wurde die Reaktionsmischung bei 60°C filtriert (m_{Ru}(FK) = 0,378 g) und mit 25 g Vinylacetat gewaschen, wonach man 85,5 g eines rot-braunen Filtrats mit einem Ru-Gehalt von 0,53 Gew.-% erhielt.

### Beispiel 4:

### Herstellung einer Katalysatorlösung mit hoher Ru-Konzentration (> 0,5 Gew.-%) und einem Vinylester/Säure-Verhältnis von 1 : 1

In einem 100 ml Berghoff-Autoklav wurden 50,0 g (0,25 mol) Laurinsäure, 1,07 g (27 mmol) Natriumhydroxid, 0,07 g Phenothiazin gegeben und 1 Stunde lang auf 120°C erhitzt, sodass entstehendes Reaktionswasser entweichen konnte. Anschließend wurden 2,36 g (8,4 mmol) Ruthenium(III)chlorid-Hydrat zugegeben und eine weitere halbe Stunde auf 120°C erhitzt, sodass Wasser entweichen konnte. Nach Zugabe von 21,5 g (0,25 mol) Vinylacetat bei 60°C wurde die Mischung 12 Stunden bei 4,9 bar abs. auf 120°C erhitzt. Nach dem Abkühlen wurde die Reaktionsmischung bei 60°C filtriert (m_{Ru}(FK) = 0,013 g) und mit 25 g Vinylacetat gewaschen, wonach man 88,4 g eines rot-braunen Filtrats mit einem Ru-Gehalt von 1,10 Gew.-% erhielt.

### Beispiel 5:

### Herstellung einer Katalysatorlösung mit hoher Ru-Konzentration (> 0,5 Gew.-%) und einem Vinylester/Säure-Verhältnis von 1 : 1

In einem 100 ml Berghoff-Autoklav wurden 50,0 g (0,25 mol) Laurinsäure, 1,73 g (43 mmol) Natriumhydroxid, 0,07 g Phenothiazin gegeben und 1 Stunde lang auf 120°C erhitzt, sodass entstehendes Reaktionswasser entweichen konnte. Anschließend wurden 3,80 g (13,5 mmol) Ruthenium(III)chlorid-Hydrat zugegeben und eine weitere halbe Stunde auf 120°C erhitzt, sodass Wasser entweichen konnte. Nach Zugabe von 21,5 g (0,25 mol) Vinylacetat bei 60°C wurde die Mischung 12 Stunden bei 4,9 bar abs. auf 120°C erhitzt. Nach dem Abkühlen wurde die Reaktionsmischung bei 60°C filtriert (m_{Ru}(FK) = 0,258 g) und mit 25 g Vinylacetat gewaschen, wonach man 65,9 g eines rot-braunes Filtrats mit einem Ru-Gehalt von 1,76 Gew.-% erhielt.

| | Ru-Konzentration[Gew.-%] | | Ru-Ausbeute [%] |
|---|---|---|---|
| | Anfang* | nach Waschen | |
| Vergleichsbeispiel 1 | 0,3 | 0,24 | 89 |
| Vergleichsbeispiel 2 | 0,3 | 0,28 | 93 |
| Vergleichsbeispiel 3 | 1,2 | 0,53 | 55 |
| Beispiel 4 | 1,2 | 1,10 | 99 |
| Beispiel 5 | 1,9 | 1,76 | 82 |

| | | | |
|---|---|---|---|
| *bezogen auf die Summe aus Laurinsäure und Vinylacetat | | | |

Vergleichsbeispiel 1 und Vergleichsbeispiel 2 zeigen, dass bei Ru-Konzentrationen von kleiner 0,5 Gew.-% die Erniedrigung des Vinylacetat/Laurinsäure-Verhältnisses zu einer erhöhten Ru-Ausbeute führt. Noch drastischer zeigt sich dieser Effekt bei Erhöhung der Ru-Konzentration. In Vergleichsbeispiel 3 werden im Vergleich zu Beispiel 4 nur 55 Gew.-% Ru-Ausbeute erhalten.

### Beispiel 6:

### Verwendung der aktiven Ru-Katalysatorlösung mit einem Ru-Gehalt > 0,5 Gew.-% in der Umvinylierung von Laurinsäure mit Vinylacetat.

In einem 100 ml Berghoff-Autoklav wurden 25,0 g Laurinsäure, 43,0 g Vinylacetat und 2,81 g Ru-Katalysatorlösung aus Beispiel 4 (1,10 Gew.-% Ruthenium) 3 Stunden lang bei 3 bar abs. auf 140°C erhitzt. Nach dem Abkühlen wurde die Reaktionsmischung mittels quantitativer NMR-Spektroskopie analysiert. Dabei konnte eine Vinyllaurat-Ausbeute von 78 % erreicht werden.

Das Beispiel zeigt, dass die erfindungsgemäße Ru-Katalysatorlösung in der Umvinylierung von Carbonsäuren eingesetzt werden kann.

## Patentansprüche

1. Verfahren zur Darstellung einer aktiven Ruthenium-Katalysatorlösung, zur Umvinylierung eines Eduktvinylesters mit einer Eduktcarbonsäure, mit einer Ru-Konzentration größer als 0,5 Gew.-%, Ru-Metall bezogen auf das Gesamtgewicht der Ruthenium-Katalysatorlösung, **dadurch gekennzeichnet, dass**
a) mindestens ein Ruthenium(III)halogenid mit mindestens einer anorganischen oder organischen Base, mit mindestens einem Eduktvinylester und mit mindestens einer Eduktcarbonsäure bei einer Temperatur von 70°C bis 170°C umgesetzt wird, wobei
b) das molare Verhältnis von Eduktvinylester zu Eduktcarbonsäure 1 : 1,8 bis 1,8 : 1 beträgt, und
c) das Ruthenium(III)halogenid in einer Menge von ≥ 0,5 Gew.-% an Ru-Metall bezogen auf das Gesamtgewicht von Eduktvinylester und Eduktcarbonsäure eingesetzt wird.

2. Verfahren nach Anspruch 1, **dadurch gekennzeichnet, dass** das Ruthenium(III)halogenid in einer Menge von 0,75 Gew.-% bis 3 Gew.-% an Ru-Metall bezogen auf das Gesamtgewicht von Eduktvinylester und Eduktcarbonsäure eingesetzt wird.

3. Verfahren nach Anspruch 1 oder 2, **dadurch gekennzeichnet, dass** als Eduktvinylester ein Carbonsäurevinylester der allgemeinen Formel R-C(O)O-CH=CH₂ eingesetzt wird, wobei R ein aliphatischen Rest mit 1 bis 12 C-Atomen sein kann, oder ein cycloaliphatischer Rest mit bis zu 12 C-Atomen sein kann, oder ein aromatischer Rest mit bis zu 12 C-Atomen sein kann.

4. Verfahren nach Anspruch 1 bis 3, **dadurch gekennzeichnet, dass** als Eduktcarbonsäure eine Carbonsäure der allgemeinen Formel R'-COOH eingesetzt wird, wobei R' ein aliphatischer Rest mit 1 bis 22 C-Atomen sein kann, oder ein cycloaliphatischer Rest mit bis zu 22 C-Atomen sein kann, oder ein aromatischer Rest mit bis zu 22 C-Atomen sein kann.

5. Verfahren nach Anspruch 1 bis 4, **dadurch gekennzeichnet, dass** molare Verhältnis von Eduktvinylester zu Eduktcarbonsäure von 1,5 : 1 bis 1 : 1 beträgt.

6. Verfahren nach Anspruch 1 bis 5, **dadurch gekennzeichnet, dass** Base und Eduktcarbonsäure bei einer Temperatur von 80°C bis 160°C, und einem Druck von ≤ 1 bar abs. vorbehandelt werden, anschließend Ru(III)halogenid zugegeben wird, und die Reaktion unter gleichen Druck- und Temperaturbedingungen fortgesetzt wird, anschließend Eduktvinylester zugegeben wird, und die Reaktion bei einer Temperatur von 70°C bis 170°C und einem Druck von > 1 bar abs. fortgesetzt wird.

## Claims

1. Method for the preparation of an active ruthenium catalyst solution, for the transvinylation of a reactant vinyl ester with a reactant carboxylic acid, having a ruthenium concentration greater than 0.5% by weight of Ru metal, based on the total weight of the ruthenium catalyst solution, **characterized in that**
a) at least one ruthenium(III) halide is reacted with at least one inorganic or organic base, at least one reactant vinyl ester and at least one reactant carboxylic acid, at a temperature of 70°C to 170°C, wherein
b) the molar ratio of reactant vinyl ester to reactant carboxylic acid is from 1:1.8 to 1.8:1, and
c) the ruthenium(III) halide is used in an amount of ≥ 0.5% by weight of Ru metal based on the total weight of reactant vinyl ester and reactant carboxylic acid.

2. Method according to Claim 1, **characterized in that** the ruthenium(III) halide is used in an amount of 0.75% by weight to 3% by weight of Ru metal, based on the total weight of reactant vinyl ester and reactant carboxylic acid.

3. Method according to Claim 1 or 2, **characterized in that** a carboxylic vinyl ester of the general formula R-C(O)O-CH=CH₂ is used as reactant vinyl ester, where R may be an aliphatic residue having 1 to 12 carbon atoms, or may be a cycloaliphatic residue having up to 12 carbon atoms, or may be an aromatic residue having up to 12 carbon atoms.

4. Method according to Claim 1 to 3, **characterized in that** a carboxylic acid of the general formula R'-COOH is used as reactant carboxylic acid, where R' may be an aliphatic residue having 1 to 22 carbon atoms, or may be a cycloaliphatic residue having up to 22 carbon atoms, or may be an aromatic residue having up to 22 carbon atoms.

5. Method according to Claim 1 to 4, **characterized in that** the molar ratio of reactant vinyl ester to reactant carboxylic acid is from 1.5:1 to 1:1.

6. Method according to Claim 1 to 5, **characterized in that** the base and reactant carboxylic acid are pretreated at a temperature of 80°C to 160°C and a pressure of ≤ 1 bar abs., then Ru(III) halide is added and the reaction is continued under the same pressure and temperature conditions, then reactant vinyl ester is added and the reaction continued at a temperature of 70°C to 170°C and a pressure of > 1 bar abs.

## Revendications

1. Procédé de préparation d'une solution active de catalyseur de ruthénium, pour la transvinylation d'un ester de vinyle réactif avec un acide carboxylique réactif, ayant une concentration en Ru supérieure à 0,5 % en poids de métal Ru par rapport au poids total de la solution de catalyseur de ruthénium, **caractérisé en ce que**
a) au moins un halogénure de ruthénium (III) est mis en réaction avec au moins une base inorganique ou organique, avec au moins un ester de vinyle réactif et avec au moins un acide carboxylique réactif à une température de 70 °C à 170 °C,
b) le rapport molaire entre l'ester de vinyle réactif et l'acide carboxylique réactif étant de 1:1,8 à 1,8:1, et
c) l'halogénure de ruthénium (III) étant utilisé en une quantité ≥ 0,5 % en poids de métal Ru par rapport au poids total de l'ester de vinyle réactif et de l'acide carboxylique réactif.

2. Procédé selon la revendication 1, **caractérisé en ce que** l'halogénure de ruthénium (III) est utilisé en une quantité de 0,75 % en poids à 3 % en poids de métal Ru par rapport au poids total de l'ester de vinyle réactif et de l'acide carboxylique réactif.

3. Procédé selon la revendication 1 ou 2, **caractérisé en ce qu'**un ester de vinyle d'acide carboxylique de la formule générale R-C(O)O-CH=CH₂ est utilisé en tant qu'ester de vinyle réactif, R pouvant être un radical aliphatique de 1 à 12 atomes C, ou pouvant être un radical cycloaliphatique de jusqu'à 12 atomes C, ou pouvant être un radical aromatique de jusqu'à 12 atomes C.

4. Procédé selon les revendications 1 à 3, **caractérisé en ce qu'**un acide carboxylique de la formule générale R'-COOH est utilisé en tant qu'acide carboxylique réactif, R' pouvant être un radical aliphatique de 1 à 22 atomes C, ou pouvant être un radical cycloaliphatique de jusqu'à 22 atomes C, ou pouvant être un radical aromatique de jusqu'à 22 atomes C.

5. Procédé selon les revendications 1 à 4, **caractérisé en ce que** le rapport molaire entre l'ester de vinyle réactif et l'acide carboxylique réactif est de 1,5:1 à 1:1.

6. Procédé selon les revendications 1 à 5, **caractérisé en ce que** la base et l'acide carboxylique réactif sont prétraités à une température de 80 °C à 160 °C, et à une pression ≤ 1 bar abs., puis l'halogénure de Ru(III) est ajouté, et la réaction est poursuivie dans les mêmes conditions de pression et de température, puis l'ester de vinyle réactif est ajouté, et la réaction est poursuivie à une température de 70 °C à 170 °C et à une pression > 1 bar abs.
